(19)

**Europäisches Patentamt**
**European Patent Office**
**Office européen des brevets**

(11) **EP 1 404 217 B1**

(12) **EUROPÄISCHE PATENTSCHRIFT**

(45) Veröffentlichungstag und Bekanntmachung des Hinweises auf die Patenterteilung:
**08.09.2010 Patentblatt 2010/36**

(21) Anmeldenummer: **02753897.4**

(22) Anmeldetag: **05.07.2002**

(51) Int Cl.:
***A61B 5/00*** *(2006.01)*

(86) Internationale Anmeldenummer:
**PCT/AT2002/000197**

(87) Internationale Veröffentlichungsnummer:
**WO 2003/003911 (16.01.2003 Gazette 2003/03)**

(54) **MESSUNG DER KONZENTRATION VON SUBSTANZEN IN LEBENDEN ORGANISMEN MITTELS MIKRODIALYSE**

MEASUREMENT OF THE CONCENTRATION OF SUBSTANCES IN LIVING ORGANISMS USING MICRODIALYSIS

MESURAGE DE LA CONCENTRATION DE SUBSTANCES DANS DES ORGANISMES VIVANTS PAR MICRODIALYSE

(84) Benannte Vertragsstaaten:
**AT BE BG CH CY CZ DE DK EE ES FI FR GB GR IE IT LI LU MC NL PT SE SK TR**

(30) Priorität: **06.07.2001 AT 10572001**

(43) Veröffentlichungstag der Anmeldung:
**07.04.2004 Patentblatt 2004/15**

(73) Patentinhaber:
• **Schaupp, Lukas**
**8010 Graz (AT)**
• **Pieber, Thomas**
**8036 Graz (AT)**

(72) Erfinder:
• **Schaupp, Lukas**
**8010 Graz (AT)**

• **Pieber, Thomas**
**8036 Graz (AT)**

(74) Vertreter: **Sonn & Partner Patentanwälte**
**Riemergasse 14**
**1010 Wien (AT)**

(56) Entgegenhaltungen:
**EP-A- 1 072 222      WO-A-96/35369**
**WO-A-99/39629      WO-A-99/45982**
**US-A- 5 298 022      US-A- 5 462 645**

• **ALCOCK S J ET AL: "CONTINUOUS ANALYTE MONITORING TO AID CLINICAL PRACTICE" IEEE ENGINEERING IN MEDICINE AND BIOLOGY MAGAZINE, IEEE INC. NEW YORK, US, Bd. 13, Nr. 3, 1. Juni 1994 (1994-06-01), Seiten 319-325, XP000456204 ISSN: 0739-5175**

## Beschreibung

Die Erfindung betrifft ein Verfahren zur Messung der Konzentration von Substanzen in lebenden Organismen mittels Mikrodialyse, gemäß dem einleitenden Teil von Anspruch 1.

Weiters bezieht sich die Erfindung auf eine Vorrichtung zur Messung der Konzentration von Substanzen in lebenden Organismen mittels Mikrodialyse, gemäß dem einleitenden Teil von Anspruch 7.

In vielen Bereichen der Medizin bzw. artverwandten Fachgebieten ist es häufig notwendig, Konzentrationen bzw. Zusammensetzungen von Körperflüssigkeiten wiederholt oder kontinuierlich zu messen, z.B. um Entgleisungen der Homöostase feststellen und behandeln zu können. So stellt Diabetes mellitus eine Entgleisung des Stoffwechsels dar, zu deren Therapie Insulin verwendet wird. Dabei können jedoch Spätkomplikationen, wie z.B. frühzeitiges Erblinden, Herz- und Nierenversagen oder Neuropathien, nicht vermieden, sondern nur verzögert werden. Eine der Schwachstellen bei der Behandlung und somit eine Ursache für die Spätfolgen dieser Erkrankung ist sicherlich die häufig nicht optimale Abstimmung von Insulininjektionen zur Blutglukose. Um die Insulininjektionen dem Bedarf des Körpers entsprechend anpassen zu können, muss zuvor die Glukosekonzentration bestimmt werden. Optimal für die richtigen Insulininjektionen wäre daher das kontinuierliche bzw. ständige Messen der Glukosekonzentration. Hierbei wurde in den letzten Jahren verstärkt Augenmerk auf die Quantifizierung der Glukose in der Gewebsflüssigkeit gelegt, welche einen engen Zusammenhang mit der Plasmaglukose hat. Probleme, welche bei der Messung im Blut auftreten, wie z.B. Gerinnung, Infektionsgefahr, Proteinbelastung usw., sind dann zumindest stark reduziert.

Im Einzelnen wurden für die kontinuierliche Messung der Glukose oder ganz allgemein von Substanzen in Gewebsflüssigkeit bereits verschiedene Möglichkeiten vorgeschlagen:

1. minimal invasive Sampling-Methoden, wie die Mikrodialyse (vgl. z.B. US 5,191,900 A; US 4,694,832 A), die Offene Mikroperfusionstechnik (vgl. z.B. EP 300 008 B, US 5,193,545 A) oder die Ultrafiltrationstechnik (s. z.B. US 5,002,054 A);

2. Sensoren, welche direkt in das Gewebe eingebracht werden (s. z.B. US 5,954,643 A); oder

3. Techniken, mit welchen die Gewebsflüssigkeit durch die Haut hindurch gesammelt werden (Suction Technik, inverse Iontophorese).

Der Mikrodialyse - auf die sich die Erfindung im Speziellen bezieht - und der Offenen Mikroperfusionstechnik gemeinsam ist das Perfundieren des Katheters mit einer Spülflüssigkeit, welche sich bei der Offenen Mikroperfusionstechnik über offene Perforationen mit der Köperflüssigkeit vermischt, wogegen bei der Mikrodialyse ein Flüssigkeitsaustausch über eine Membran stattfindet. Diese Membran hat den Vorteil, dass der Austausch von Molekülen zwischen Körper- und Spülflüssigkeit selektiv gesteuert werden kann (Größe, Form, Ladung etc. der Moleküle), jedoch werden diese Eigenschaften durch Ablagerungen von endogenen Substanzen (vorwiegend Proteine, aber auch Zellen) während der Zeit verändert. Diese Ablagerung geht mit einer Veränderung der Transporteigenschaften der Moleküle über die Membran einher, was sich in einer verminderten Konzentration der Moleküle in der Spülflüssigkeit widerspiegelt.

Gemäß der EP 0 300 008 B wird diese Ablagerung derart umgangen, dass die als Subkutannadel/-katheter ausgeführte Hohlnadel im Wandbereich einen offenen Austauschkanal aufweist, so dass ein offener Austausch zwischen der Körper- und der Spülflüssigkeit stattfinden kann, d.h. diese Methode gehört zur offenen Mikroperfusionstechnik.

Die WO 96/35369 A1 beschreibt ein Verfahren zur Bestimmung der Produktion von bestimmten Körpersubstanzen, bei dem eine Kalibrierung durch Quotientenbildung vorgenommen wird. Dabei wird der zu untersuchenden Substanz eine Kalibrationsflüssigkeit beigefügt, wobei die Konzentration und Menge der Kalibrationssubstanz bekannt sind. Unter der Voraussetzung, dass die Kalibrationsflüssigkeit nicht abgebaut wird, kann die relative Produktionsrate der zu untersuchenden Substanz bestimmt werden, nicht jedoch die absolute Konzentration.

Die DE 197 15 440 C1 beschreibt eine - ähnliche - Technik, bei der eine Konditionier- und Standardlösung von extern zugeführt wird. Da bei dieser Anordnung keine Quantifizierung der zu bestimmenden Substanz möglich ist, werden Vergleiche vorgenommen, und durch Extrapolation der Signale kann die Konzentration der zu bestimmenden Substanz eruiert werden. Die Anordnung ist besonders aufwendig, da sowohl die Konzentration als auch die Durchflussgeschwindigkeit der Konditionier- und Standardlösung verändert werden müssen.

Die EP 1 072 222 A2 beschreibt ein Verfahren und eine Anordnung zur Konzentrationsbestimmung von Glucose in einer Körperflüssigkeit, bei dem ein Vergleich zwischen einer extern zugeführten Perfusatlösung und der Konzentration der Substanz im Körper durchgeführt wird. Dabei werden von außen verschiedene Glucosekonzentrationen dem Perfusat beigemengt. Neben der Komplexität der Anordnung ist das System auch besonders träge, da die externe Zugabe von Glukose der körpereigenen Glukosekonzentration immer nachgeführt werden muss.

Die WO 93/05701 sowie die GB 2 297 383 A betreffen Elektrodenanordnungen, die in einem Mikrodialysekatheter untergebracht sind. Dabei geht es um die Messung der elektrischen Leitfähigkeit und nicht um die Messung der Konzentration einer bestimmten Substanz.

Die Erfindung sucht nun die Vorzüge der Membran (sterile Barriere, gezielter Ausschluss von Molekülen, wie z.B. Proteinen, etc.) zu nützen und dabei die Ablagerungen an der Membran zu berücksichtigen, wobei die Erfindung danach trachtet, ein Quantifizieren der Durchlässigkeit der Membran während der Messung zu erreichen.

Es ist demgemäß Aufgabe der Erfindung, ein Verfahren sowie eine Vorrichtung wie eingangs angeführt vorzuschlagen, um eine zuverlässige Messung der Konzentration der gewünschten Substanzen trotz Verwendung einer Membran im Zuge der Mikrodialyse-Technik sicherzustellen und dabei gleichzeitig die Vorteile der Verwendung einer Membran, wie die Bereitstellung einer sterilen Barriere, eines gezielten Ausschlusses oder Durchlasses von Molekülen usw., zu nützen.

Zur Lösung dieser Aufgabe sieht die Erfindung ein Verfahren sowie eine Vorrichtung wie in den unabhängigen Ansprüchen definiert vor.

In den abhängigen Ansprüchen sind vorteilhafte Ausgestaltungen und Weiterbildungen der Erfindung gekennzeichnet.

Mit der erfindungsgemäßen Technik werden die Vorteile der Verwendung der Mikrodialyse, d.h. des Einsatzes einer Membran, mit jenen einer genauen Bestimmung der Konzentration der gewünschten Substanzen aufgrund der Verwendung von Referenzsubstanzen vereinigt. Dabei werden Referenzionen herangezogen, deren Konzentration im Gewebe bekannt und konstant ist.

Die Equilibration (der Austausch) zwischen Köperflüssigkeit und Spülflüssigkeit (Perfusat) ist eine Funktion der Membran-Austauschfläche und der Fließgeschwindigkeit der Spülflüssigkeit. Bei unendlich geringer Fließgeschwindigkeit findet eine vollständige Equilibration zwischen beiden Flüssigkeiten statt. Aufgrund der langsamen Fließgeschwindigkeit ergeben sich aber für die Messung der Substanzen in der Spülflüssigkeit zwei entscheidende Nachteile: Erstens ist die gewonnene Flüssigkeitsmenge pro Zeiteinheit sehr gering; und zweitens wird die Verzögerung aufgrund der Schlauchlänge (Systemverzögerung) entsprechend groß.

Aus diesem Grund ist eine höhere Fließgeschwindigkeit gewünscht, um mehr Flüssigkeit schneller zur Verfügung zu haben. Der Nachteil dieser Betriebsart besteht in der nicht vollständigen Vermischung der beiden Flüssigkeiten, was durch Messungen anderer Parameter ausgeglichen werden könnte, wodurch sich zusätzliche Anforderungen an die Messtechnik ergeben, was sich besonders bei online-Messungen als Schwierigkeit entpuppt. Um die tatsächliche Wiederfindungsrate (Recovery) und damit die Konzentration außerhalb des Katheters quantifizieren zu können, wurden verschiedene Kalibrationsmethoden für Samplingtechniken vorgeschlagen: Die einfachste Methode besteht in der Bestimmung der Wiederfindungsrate in vitro. Mit dieser gefundenen Rate werden dann die in vivo gemessenen Werte korrigiert. Es konnte vielfach gezeigt werden, dass sich die Wiederfindungsrate in vitro und in vivo nicht nur unterscheiden, sondern dass sich die Wiederfindungsrate in vivo zeitlich ändert.

Eine weitere Methode zur Bestimmung der Wiederfindungsrate besteht im Verändern der Fließgeschwindigkeit und einer damit verbundenen Veränderung der Wiederfindungsrate. Ist der funktionale Zusammenhang zwischen Wiederfindungsrate und Fließgeschwindigkeit bekannt, kann auf die Konzentration außerhalb des Katheters rückgerechnet werden (diese Bestimmung der Wiederfindungsrate wird in der Literatur "Zero Flow Rate"-Protokoll genannt, weil bei der Fließgeschwindigkeit "Null" eine vollständige Vermischung zwischen der Flüssigkeit außerhalb des Katheters und der Spülflüssigkeit stattfindet). Diese Bestimmung ist zeitaufwendig und technisch relativ aufwendig umzusetzen, weil die Fließgeschwindigkeit permanent geändert werden muss.

Das "No-Net-Flux"-Protokoll stellt eine weitere Möglichkeit dar, die Wiederfindungsrate und damit die Konzentration außerhalb des Katheters zu bestimmen. Dabei werden der Spülflüssigkeit unterschiedliche Konzentrationen der zu messenden Substanz zugesetzt. Durch die sich dabei ergebenden Änderungen der Konzentrationen am Ausgang des Katheters kann auf die Konzentration im Körper bzw. auf die Wiederfindungsrate geschlossen werden.

Alle bisher erwähnten Kalibrationsmethoden sind jedoch nicht in der Lage, sich ändernde wiederfindungsraten während einer in vivo-Messung zu bestimmen. Zur Bestimmung der dynamischen Wiederfindungsrate werden daher erfindungsgemäß körpereigene Referenzionen mitgemessen. Dabei ist es notwendig, dass die Wiederfindungsraten für die zu bestimmende Substanz und für die Referenzionen proportional sind. Durch einen Konzentrationsunterschied zwischen der Spülflüssigkeit und den Referenzionen kommt es zu einem Nettofluss zwischen den Referenzionen im Katheter und der den Katheter umgebenden zu messenden Flüssigkeit. Dieser Nettofluss spiegelt sich in einer Differenz der Konzentration der Spülflüssigkeit, welche in den Katheter hinein- und herausfließt, wider.

Die Erfindung nützt somit das Vorhandensein von endogenen Referenzionen in der Flüssigkeit, in welcher die Konzentration bestimmt werden soll, also in der Körperflüssigkeit. In der Medizin werden dazu häufig sogenannte Tracer eingesetzt, welche in den Organismus eingebracht werden (exogene Referenz). Für wissenschaftliche Untersuchungen ist es vertretbar, diese zum Teil belastenden (z.B. radioaktiv) Substanzen einzubringen. Für die Entwicklung eines Systems, welches routinemäßig von Patienten eingesetzt werden soll, erscheint es dagegen notwendig, Referenzsubstanzen heranzuziehen, welche bereits im Organismus vorkommen (endogene Referenz). Für die Offene Mikroperfusionstechnik (EP 0 300 008 B; US 5,193,545 A; US 5,097,834 A), bei welcher im Unterschied zur Erfindung ein direkter Kontakt zwischen Körperflüssigkeit und Spülflüssigkeit stattfindet, wird die Osmolarität, Impedanz oder elektrische Leitfähigkeit als Maß für die wiederfindungsrate vorgeschlagen.

Die vorliegende Erfindung basiert wie erwähnt auf der Mikrodialysetechnik für die Gewinnung von Körperflüssigkeiten. Dabei wird die Wiederfindungsrate bevorzugt mit Hilfe der im Körper sehr konstant gehaltenen Ionen bzw. der damit verbundenen elektrischen Leitfähigkeit oder Osmolarität unter der Berücksichtigung der unterschiedlichen Diffusionsgeschwindigkeiten über die Mikrodialyse-Membran bestimmt. über die Bestimmung der Wiederfindungsrate aufgrund des eingeschränkten Transportes von Molekülen im Gewebe selbst und des Austauschvorgangs zwischen Körperflüssigkeit und der Spülflüssigkeit hinaus ist es möglich, durch die Bestimmung von Ionen bzw. der damit verbundenen elektrischen Leitfähigkeit Ablagerungen an der Mikrodialyse-Membran selbst zu berücksichtigen. Das stellt einen wesentlichen Unterschied zu der vorhin erwähnten Offenen Mikroperfusionstechnik dar, bei welcher mit Hilfe von Osmolarität, Impedanz oder elektrischer Leitfähigkeit nur der eingeschränkte Transport im Gewebe selbst charakterisiert wird, weil durch das

Nichtvorhandensein einer Membran keine weitere Transportbarriere entsteht. Demgegenüber werden bei der Mikrodialyse durch die Messung von Referenzionen auch die eingeschränkten bzw. sich ändernden Transporteigenschaften über die Membran berücksichtigt.

Von vorteil ist es bei der erfindungsgemäßen Technik auch, wenn ein etwaiges unterschiedliches Transportverhalten von Referenzionen und zu messender Substanz durch einen vorab bestimmten Korrekturfaktor berücksichtigt wird. Der genannte Korrekturfaktor kann dabei vorab in vitro dadurch bestimmt werden, dass eine Lösung mit Ionen und der zu bestimmenden Substanz angesetzt und diese Lösung durch die Membran oder eine vergleichbare Membran hindurch geleitet, z.B. angesaugt wird. Möglich ist es aber auch, zur Bestimmung dieses Korrekturfaktors eine Parallelmessung der Substanz im Blut vorzunehmen und den Messwert im Blut zum Messwert im Gewebe in Beziehung zu setzen, um so den Korrekturfaktor zu erhalten.

Im Rahmen der Erfindung wird die Messung der zu ermittelnden Größe bevorzugt auf Basis der Messung der elektrischen Leitfähigkeit entsprechend der Summe von Ionen vorgenommen.

Um hohe Durchflussraten der Spülflüssigkeit bzw. des Gemischs Spülflüssigkeit/Körperflüssigkeit zu erzielen, kann eine Zwangsförderung mit Hilfe von Pumpeinheiten vorgesehen werden. Vorzugsweise wird dabei im Zulauf zum Mikrodialyse-Katheter ebenso wie im Ablauf von diesem eine Pumpeinheit angeordnet, und die beiden Pumpeinheiten werden vorzugsweise synchronisiert, um einen gleichmäßigen Flüssigkeitstransport zu gewährleisten. Um die jeweilige Flüssigkeit so rasch wie möglich vom Katheter zum Sensor zu bringen, ist zweckmäßigerweise die Ablauf-seitige Pumpeinheit in Strömungsrichtung gesehen hinter der Sensoreinheit angeordnet. Die Pumpeinheiten können mit Vorteil einfach durch Peristaltikpumpen gebildet sein.

Wie erwähnt werden als Referenzgröße Ionen verwendet, wobei die Leitfähigkeit bestimmt wird. Demgemäß enthält die Sensoreinheit vorteilhafterweise Elektroden zur Bestimmung der Leitfähigkeit der durchströmenden Flüssigkeit.

Die Erfindung wird nachfolgend anhand von bevorzugten Ausführungsbeispielen unter Bezugnahme auf die Zeichnung noch weiter erläutert.

Es zeigen:

Fig.1 schematisch die Zusammensetzung von menschlicher Gewebsflüssigkeit unter spezieller Veranschaulichung der Kationen und Anionen darin;
Fig.2 ein Diagramm zur Veranschaulichung der Wiederfindungsrate von Ionen und dazugehörig von der elektrischen Leitfähigkeit einer Gewebsflüssigkeit;
Fig.3 schematisch in einem Diagramm die Konzentrationsverläufe von Glukose und Natrium proportional zur elektrischen Leitfähigkeit;
Fig.4 in einer Art Blockschaltbild eine Vorrichtung mit einem in ein Körpergewebe einzubringenden Mikrodialyse-Katheter, zur Durchführung des erfindungsgemäßen Messverfahrens;
Fig.5 eine mögliche Ausführung der bei einer solchen Vorrichtung verwendeten Pumpeinheiten in Form von zwei gesonderten Peristaltikpumpen;
Fig.6 eine kombinierte Peristaltikpumpe zur Bildung der bei der Vorrichtung gemäß Fig.4 vorhandenen Pumpeinheiten;
Fig.7 eine Ausführungsform des Perfusat-Reservoirs für die Vorrichtung gemäß Fig.4;
Fig.8 - und in einem vergrößerten Detail in Fig.8A - einen Mikrodialyse-Katheter;
Fig.9 schematisch eine Ausführungsform eines Sammelgefäßes für eine Vorrichtung gemäß Fig.4; und die
Fig.10 und 11 in einem schematischen Längsschnitt bzw. Querschnitt eine Sensoreinheit für die Vorrichtung gemäß Fig.4.

wie erwähnt, wird bei der vorliegenden Messtechnik zur Bestimmung der Konzentration von Substanzen in lebenden Organismen die sog. Mikrodialyse-Technik angewandt, die für sich genommen bekannt ist, jedoch wegen des Nachteils der Ablagerung von körpereigenen Substanzen an der Membran in der Vergangenheit verworfen wurde, da man dadurch die Messergebnisse als unbrauchbar ansah. Mit der hier vorgeschlagenen Technik wird jedoch eine Lösung vorgesehen, gemäß der auf der Basis von der Messung von Referenzionen eine Berücksichtigung der eingeschränkten oder sich ändernden Transportcharakteristika beim Hindurchtritt der Körperflüssigkeit durch die Membran berücksichtigt wird.

Im Einzelnen wird über diese Membran ein Austausch von Körperflüssigkeit ermöglicht, wie dies an sich bekannt ist, wobei auf der anderen Seite der Membran eine Spülflüssigkeit, ein sog. Perfusat, vorbeigeleitet wird.

Für die Charakterisierung dieses Austauschvorganges wird ein beispielsweise ionenfreies oder mit einer definierten Ionenkonzentration versetztes Perfusat eingesetzt. Die Änderung der Ionenkonzentration zwischen der Spülflüssigkeit vor und nach der Equilibration mit der Körperflüssigkeit ist ein Maß für die Wiederfindung. Dabei können einzelne Ionen selektiv gemessen werden (z.B. Natrium oder Chlorid), oder es wird bevorzugt die elektrische Leitfähigkeit als die gewichtete Summe aller Ionenkonzentrationen gemessen:

$$\chi = F \cdot \sum |z_i| \cdot c_i \cdot u_i \quad , \tag{1}$$

wobei
$\chi$ die elektrische Leitfähigkeit [$A \times V^{-1} \times m^{-1}$],
F die Faraday-Konstante [$A \times s \times mol^{-1}$],
z die Ladungszahl der Ionensorte,
c die Ionenkonzentration [mmol/l] und
u die Ionenbeweglichkeit [$m^2 \times V^{-1} \times s^{-1}$] ist.

Der Berechnung für die Bestimmung der interessierenden Substanz wird folgende Annahme zugrunde gelegt: Die Wiederfindungsraten - Verhältnis einer Substanz im Rückperfusat ($Substanz_{PERF}$) eines Katheters zur Substanz im Organismus ($SubStanz_{ORC}$) - für die im Organismus zu bestimmende Substanz $Substanz_{ORG}$ und die Referenzionen $Referenz_{ORG}$ sind proportional:

$$Wiederfindungsrate = \frac{Substanz_{PERF}}{Substanz_{ORG}} = k \cdot \frac{Referenz_{PERF}}{Referenz_{ORG}} \tag{2}$$

Durch umformen dieser Gleichung 2 erhält man für die zu messende Substanz $Substanz_{ORG}$ folgende Gleichung:

$$Substanz_{ORG} = Substanz_{PERF} \cdot \frac{1}{k} \cdot \frac{Referenz_{ORG}}{Referenz_{PERF}} \tag{3}$$

mit:

| | |
|---|---|
| $Substanz_{ORG}$ | zu bestimmende Substanz im Organismus |
| $Substanz_{PERF}$ | zu bestimmende Substanz im Rückperfusat |
| $Referenz_{ORG}$ | Referenzionen-Konzentration im Organismus |
| $Referenz_{PERF}$ | Referenzionen-Konzentration im Rückperfusat |
| k | Korrekturfaktor für die unterschiedliche Wiederfindungsrate |

Der Korrekturfaktor k berücksichtigt das unterschiedliche Transportverhalten der Referenzionen und der zu bestimmenden Substanz. Dieser Korrekturfaktor k, welcher sich für jede zu bestimmende Substanz neu ergibt, kann auf verschiedene Weisen bestimmt werden. Eine Möglichkeit ist beispielsweise eine in vitro-Bestimmung, bei der eine Lösung mit Ionen und der zu bestimmenden Substanz angesetzt und über einen Mikrodialysekatheter gesammelt wird; durch Messung der Konzentrationen von Ionen und der zu bestimmenden Substanz im Dialysat und der bekannten Konzentrationen in der angesetzten Lösung kann der Korrekturfaktor k bestimmt werden. Andererseits kann auch eine in vivo-Bestimmung vorgenommen werden. In vielen Bereichen der Medizin stellen die Konzentrationen im Blut den "Goldenen Standard" dar, d.h. quantitatives Wissen in der Medizin wird meist auf Blut bezogen. Zur Bestimmung des Korrekturfaktors k kann nun eine Messung im Blut parallel zur Messung im Gewebe vorgenommen werden. Durch die Beziehung des Gewebewertes zum Blutwert ergibt sich ein Korrekturfaktor $k_g$, der jetzt nicht nur den Korrekturfaktor k berücksichtigt, sondern auch eventuelle Unterschiede zwischen Blut- und Gewebekonzentrationen mit berücksichtigt, d.h. der Faktor wird nicht mehr explizit bestimmt, sondern geht in den Umrechnungsfaktor zwischen Blut- und Gewebekonzentration ein.

Die Bestimmung der Konzentration der Substanz im Organismus ($Substanz_{ORC}$) basiert gemäß der obigen Beziehung (3) auf der Messung der zu bestimmenden Substanz im Rückperfusat ($Substanz_{PERF}$), der Messung der Referenzionen im Rückperfusat ($Referenz_{PERF}$), der Bestimmung des Korrekturfaktors für die unterschiedliche Wiederfindungsrate und der Annahme der konstanten Größe der Referenzionen im Organismus ($Referenz_{ORG}$).

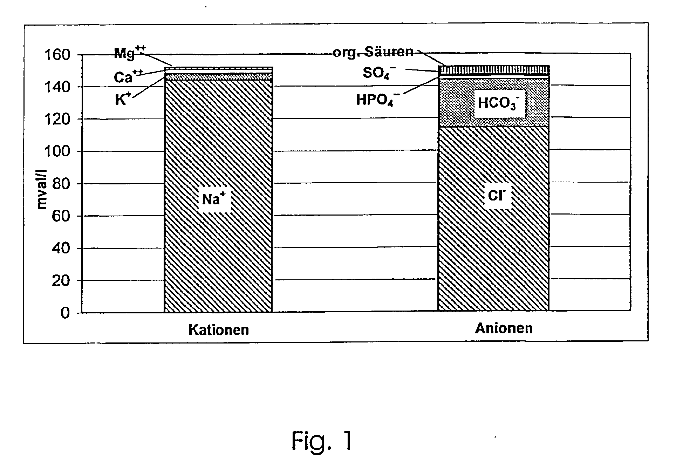

In Fig.1 ist schematisch die Zusammensetzung von menschlicher Gewebsflüssigkeit hinsichtlich Ionen dargestellt. Der Großteil der Ionen besteht wie ersichtlich aus Natrium-Ionen und Chlorid-Ionen. Wegen der Aufrechterhaltung des kolloid osmotischen Drucks werden die Konzentrationen der Elektrolyte in einem sehr kleinen Toleranzbereich aufrecht erhalten und damit auch die elektrische Leitfähigkeit, welche dadurch als endogene Referenz herangezogen wird.

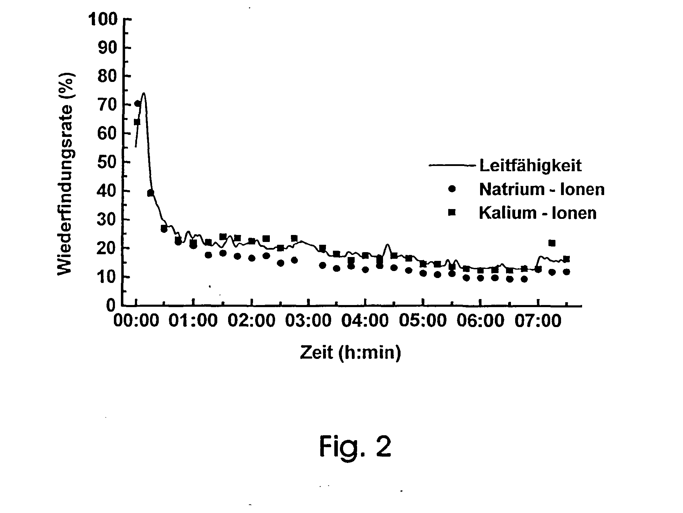

In Fig.2 ist graphisch die wiederfindungsrate von Ionen, nämlich Natrium- und Kalium-Ionen, sowie dazugehörig der elektrischen Leitfähigkeit von Gewebsflüssigkeit über der Zeit dargestellt, wobei die Ionen mit Hilfe von Mikrodialyse gewonnen wurden. Dabei ist aus dem Diagramm von Fig.2 zu erkennen, dass die Ionenkonzentration durch die elektrische Leitfähigkeit wiedergegeben werden kann. Weiters erkennt man aus diesem Humanexperiment, dass sich die wiederfindungsrate über die Zeit ändert; das kann auf Änderungen der Membrandurchlässigkeit (Ablagerungen), aber auch auf Änderungen des Transportverhaltens im Gewebe zurückzuführen sein. Für eine verlässliche Messung ist es deshalb notwendig, diese wiederfindungsrate zu jedem Zeitpunkt einer Messung bestimmen zu können.

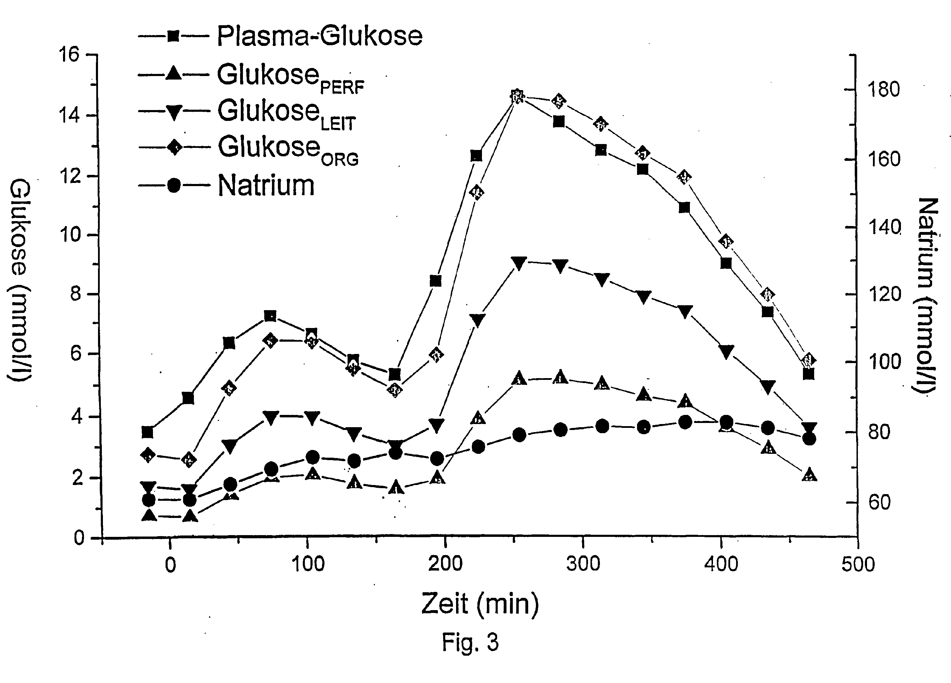

In Fig.3 sind die Konzentrationsverläufe von Glukose und Natrium (die proportional zur elektrischen Leitfähigkeit ist) dargestellt. Durch die Messung von Natrium und $Glukose_{PERF}$ (im Perfusat enthaltene Glukose) kann auf die $Glukose_{LEIT}$ (Glukosekonzentration korrigiert mit der Leitfähigkeit) rückgerechnet werden. Diese $Glukose_{LEIT}$-Konzentration wird weiter durch den Vergleich mit Plasmaglukose zu $Glukose_{ORG}$ (Glukose im Organismus) hochgerechnet und steht als Messergebnis zur Verfügung. Zu beachten ist die sich ändernde Natriumkonzentration (entspricht der elektrischen Leitfähigkeit), was eine Veränderung der Wiederfindungsrate widerspiegelt und die notwendige kontinuierliche Bestimmung dieser Größe unterstreicht.

Die nachfolgende Tabelle zeigt die Regression zwischen Plasmaglukose und Perfusatglukose ($Substanz_{PERF}$) und Plasmaglukose und der Gewebsglukose ($Substanz_{ORG}$): $y = A_x + B_x * Perfusatglukose$, mit $r_x$...linearer Korrelationskoeffizient

| | Perfusatglukose ($Substanz_{PERF}$) | | | Gewebsglukose ($Substanz_{ORG}$) | | |
|---|---|---|---|---|---|---|
| Patient | $A_1$ | $B_1$ | $r_1$ | $A_2$ | $B_2$ | $r_2$ |
| 1 | -0,44 | 0,412 | 0,875 | 0,979 | 0,769 | 0,962 |
| 2 | 0,714 | 0,312 | 0,886 | 1,039 | 0,606 | 0,963 |
| 3 | -0,879 | 0,43 | 0,961 | -0,971 | 0,708 | 0,974 |
| 4 | 2,776 | 0,237 | 0,769 | 0,489 | 0,702 | 0,992 |
| 5 | 0,284 | 0,217 | 0,654 | -0,165 | 0,651 | 0,952 |
| 6 | 0,833 | 0,19 | 0,716 | -0,647 | 0,672 | 0,962 |
| | | | | | | |

Die Gewebsglukose wurde mit Hilfe der Perfusatglukose- und Ionenkonzentration (proportional zur elektrischen Leitfähigkeit) ermittelt. In allen Fällen konnte die Korrelation zwischen Plasmaglukose und Gewebsglukose durch die Korrektur mit der Leitfähigkeitsmessung signifikant verbessert werden, was sich durch ein Ansteigen des Korrelationskoeffizienten $r_1$ auf $r_2$ (knapp unter 1) zeigen lässt.

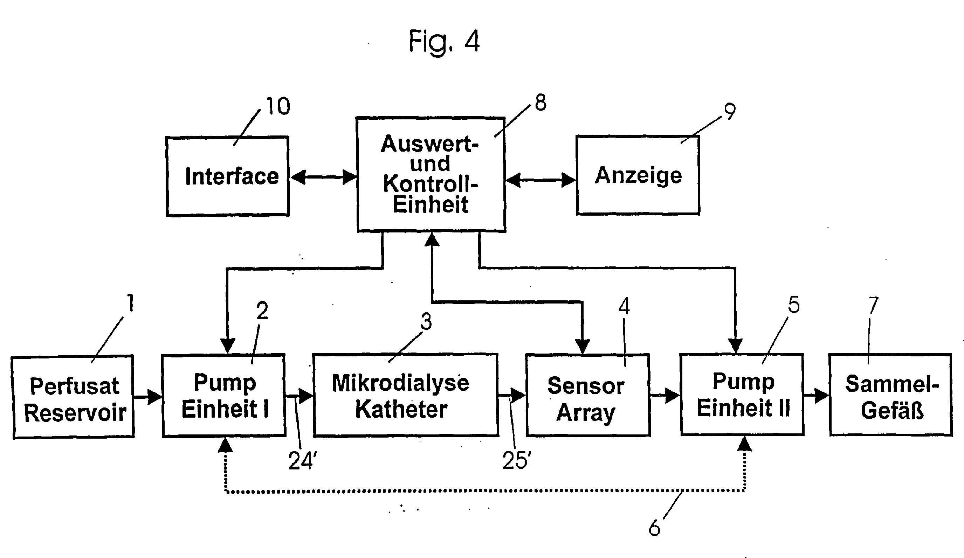

Fig.4 zeigt in einem Blockschaltbild eine Messvorrichtung nach dem beschriebenen Mikrodialyseprinzip. Aus einem Perfusatreservoir 1 wird über eine erste Pumpeinheit 2 eine Trägerflüssigkeit in einen Mikrodialyse-Katheter 3 gepumpt. Diese Trägerflüssigkeit durchströmt den Mikrodialyse-Katheter 3, vermischt sich dabei zum Teil mit Körperflüssigkeit und wird anschließend zu einer Sensoreinheit 4 geführt. Um einen gleichmäßigen Flüssigkeitstransport zu gewährleisten, befindet sich nach dem Mikrodialyse-Katheter 3 eine zweite Pumpeinheit 5, welche mit der ersten Pumpeinheit 2 synchronisiert ist, was mit einer strichlierten Linie in Fig.4 angedeutet ist. Dabei kann die zweite Pumpeinheit 5 vor oder nach der Sensoreinheit 4 positioniert sein. Um Verzögerungen durch den Transport vom Katheter 3 zur Sensoreinheit 4 so gering wie möglich zu halten, ist es von Vorteil, wenn die Sensoreinheit 4 unmittelbar nach dem Katheter 3 angebracht ist.

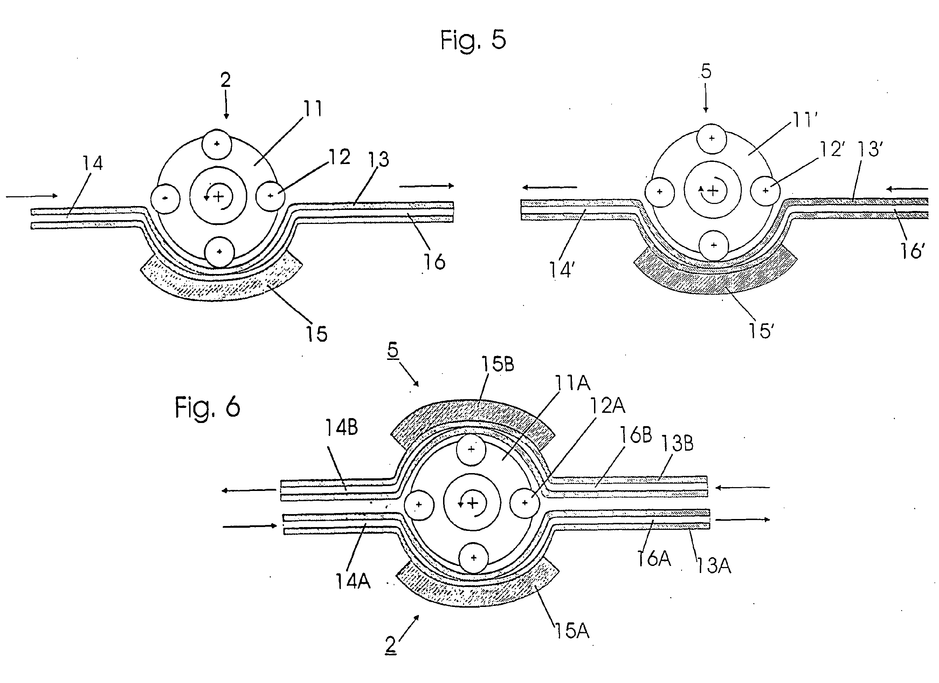

Die Pumpeinheiten 2, 5 können als einzelne Peristaltikpumpen ausgeführt sein, aber auch das Anbringen von Pump- und Saugschlauch am selben Pumpenkopf ist denkbar, vgl. die nachfolgend erläuternden Fig.5 und 6. Die Pumpeinheiten 2, 5 sind jedoch nicht auf Peristaltikpumpen beschränkt, sondern jede Art von Pumpe (Kolbenpumpe, Unter-, Überdruckgefäße, ...) ist denkbar.

Am Ende der Fluidik wird gemäß Fig.4 die Flüssigkeit in einem Sammelgefäß 7 gespeichert. Eine Auswert- und Kontrolleinheit 8 koordiniert die Flussraten der Pumpen 2, 5 und steuert eine Anzeige 9 und ein Interface 10, welches zur Eingabe von Operationen sowie zur Ausgabe von Daten dient.

In Fig.5 sind die zwei Pumpeinheiten 2, 5 als gesonderte Peristaltikpumpen dargestellt. Es handelt sich dabei um peristaltische Schlauchquetschpumpen, wobei jeweils an einem Pumpenkopf 11, 11' Rollen 12, 12' angebracht sind. Durch Drehung des Pumpenkopfes 11, 11' wird das Lumen eines zugehörigen Schlauches 13, 13' in zwei Teile unterteilt, nämlich in das Lumen 14, 14' und das Lumen 16, 16'. Der Schlauch 13, 13' wird zwischen den Rollen 12, 12' und einem Gegenstück 15, 15' gequetscht, wobei durch Drehung des Pumpenkopfes 11, 11', z.B. gegen den Uhrzeigersinn, ein Flüssigkeitstransport z.B. vom Lumen 14, 14' zum Lumen 16, 16' stattfindet. Um synchron Flüssigkeit zu pumpen und zu saugen, werden die zwei Pumpeneinheiten 2, 5 jedoch mit unterschiedlicher Drehrichtung eingesetzt, wie in Fig.5 gezeigt ist; die zweite Pumpeneinheit 5 wird demgemäß im Uhrzeigersinn betrieben.

Eine weitere Möglichkeit für die Realisierung der zwei Pumpeinheiten 2, 5 ist in Fig.6 gezeigt und besteht im gegenläufigen Anbringen von zwei Schläuchen 13A, 13B auf einem Pumpenkopf 11A mit Rollen 12A und zwei Gegenstücken 15A, 15B. Unter der Voraussetzung, dass beide Schläuche 13A, 13B und die dazugehörigen Gegenstücke 15A, 15B die gleiche Charakteristik aufweisen, ist absolute Synchronität zwischen beiden Pumprichtungen gegeben. Die durch die Pumpeinheiten 2, 5 unterteilten Schlauch-Lumina sind in Fig.6 entsprechend mit 14A, 16A bzw. 14B, 16B bezeichnet. Weiters ist die Fließrichtung in Fig.6 ebenso wie in Fig.5 jeweils mit Pfeilen angegeben.

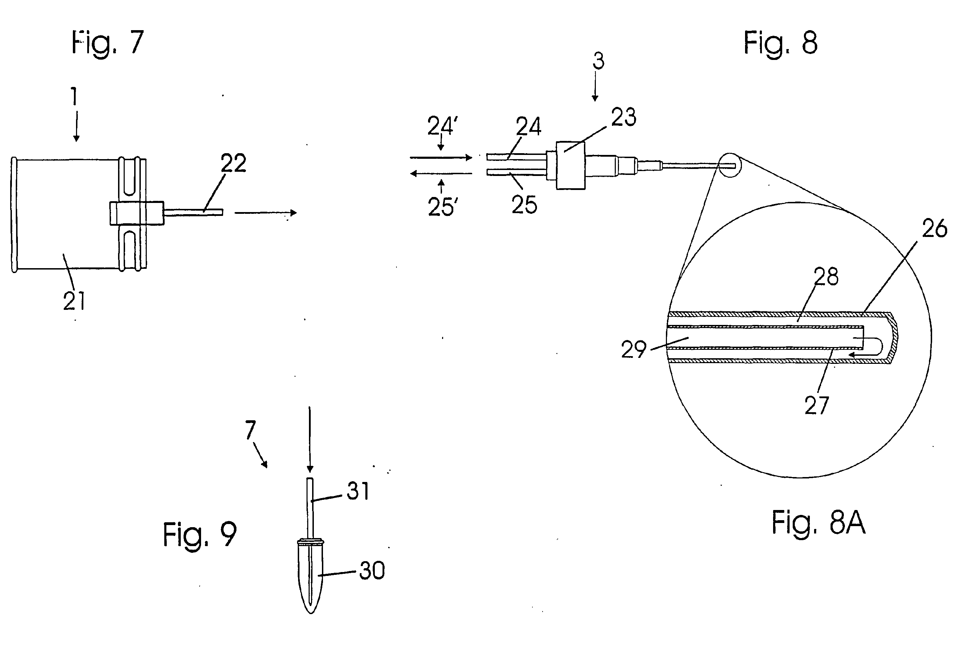

Eine mögliche Ausführungsform des Perfusatreservoirs 1 besteht gemäß Fig.7 in Form eines Kunststoffbeutels 21 mit einem Anschlussschlauch 22. Eine weitere mögliche Ausführungsform besteht in einer Ampulle.

Gemäß Fig.8 besteht der Mikrodialyse-Katheter 1 aus einem Körper 23 und zwei Anschlüssen, nämlich einem Zuführanschluss 24 und einem Abführanschluss 25. In Fig.8 wie auch in Fig.4 sind demgemäß auch allgemein der Zulauf zum Katheter 3 mit 24' und der Ablauf mit 25' bezeichnet. Der Katheter 3 kann linear ausgeführt sein, vergleichbar einem Schlauch mit einem Eingang auf der einen Seite und einem Ausgang auf der anderen Seite, vgl. z.B US 5,706,806 A, oder aber auch - wie gemäß Fig.8 - als doppellumiger Katheter, vgl. auch US 4,694,832 A. Dabei umschließt, wie in der Detaildarstellung von Fig.8A an der Katheterspitze ersichtlich, eine Membran 26 ein Rohr 27, wobei ein Lumen 28 gebildet wird. Durch das Lumen 29 des Rohres 27 wird Perfusat in dieses Zwischenraum-Lumen 28 eingebracht, wobei für die prinzipielle Funktionsweise des Katheters 3 die Fließrichtung auch umgedreht werden kann.

Gemäß Fig.9 besteht das Sammelgefäß 7 beispielsweise aus einem Behälter 30 mit einer Schlauchverbindung 31.

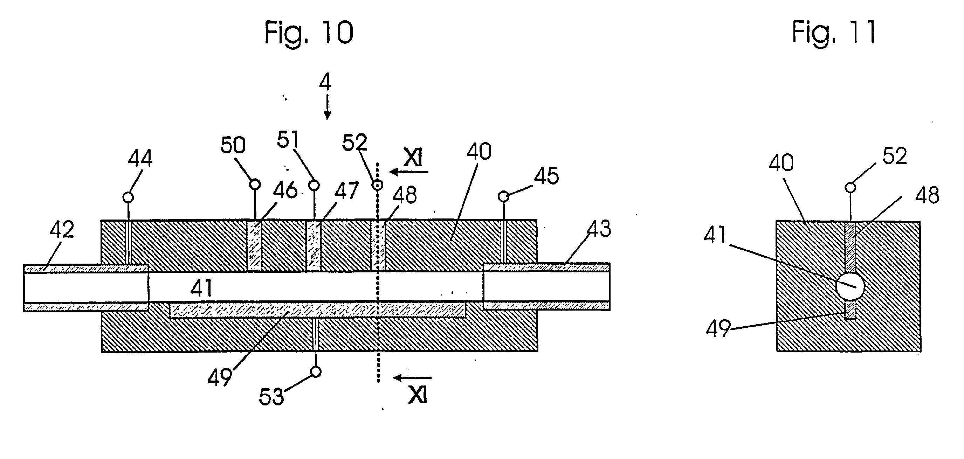

In den Fig.10 und 11 ist eine mögliche Ausführungsform der Sensoreinheit 4 dargestellt. Ein Körper 40 der Sensoreinheit 4 ist von einem Lumen 41 durchzogen, in welchem die zu analysierende Flüssigkeit geführt wird. Über Anschlussstücke 42, 43 wird dieser Sensor-Körper 40 mit den zugehörigen Komponenten (z.B. 3 bzw. 5 in Fig.4) verbunden. Diese Anschlussstücke 42, 43 können aus Metall ausgeführt sein, wobei über eine elektrische Verbindung 44, 45 zwischen diesen Anschlussstücken 42, 43 die elektrische Leitfähigkeit bestimmt werden kann (axiale Bestimmung der Leitfähigkeit). Eine andere Möglichkeit besteht in der radialen Bestimmung der Leitfähigkeit, wobei zwischen einer Elektrode 46, 47 oder 48 und einer gegenüberliegenden Elektrode 49 die Leitfähigkeit bestimmt wird. Zur Leitfähigkeitsbestimmung werden an sich nur zwei Elektroden benötigt, z.B. die Elektroden 46 und 49, wobei dann die anderen Elektroden 47 und 48 für die Bestimmung von weiteren Analyten (z.B. Glukose, Laktat, ...) herangezogen werden können. Die Kontaktierung der Elektroden 46, 47, 48 bzw. 49 erfolgt über Anschlüsse 50, 51, 52 bzw. 53.

**Patentansprüche**

1. Verfahren zur Messung der Konzentration von Substanzen in lebenden Organismen mittels Mikrodialyse, wobei Körperflüssigkeit durch eine Membran hindurch mit einer Spülflüssigkeit vermischt und eine Substanz-Konzentration der Körperflüssigkeit mit Hilfe einer Sensoreinheit bestimmt wird, **dadurch gekennzeichnet, dass** zur Berücksichtigung des eingeschränkten Transports der Substanz durch die Membran, so dass die gemessene Substanz-Konzentration von der tatsächlichen Substanz-Konzentration im Organismus verschieden ist, ergänzend als endogene Referenzsubstanz Ionen mit bekannter Konzentration nach Durchtritt durch die Membran und Vermischen mit der Spülflüssigkeit auf Basis der Messung von elektrischer Leitfähigkeit entsprechend den Referenzionen gemessen werden und die tatsächliche Substanz-Konzentration auf Basis des Quotienten aus bekannter Referenzionen-Konzentration durch gemessene Referenzionen-Konzentration, multipliziert mit der bestimmten Substanz-Konzentration ermittelt wird.

2. Verfahren nach Anspruch 1, **dadurch gekennzeichnet, dass** ein etwaiges unterschiedliches Transportverhalten von Referenzionen und zu messender Substanz durch einen vorab bestimmten Korrekturfaktor berücksichtigt wird.

3. Verfahren nach Anspruch 2, **dadurch gekennzeichnet, dass** der Korrekturfaktor vorab in vitro durch Ansetzen einer Lösung mit Ionen und der zu bestimmenden Substanz und durch Hindurchleiten durch eine Membran bestimmt wird.

4. Verfahren nach Anspruch 2, **dadurch gekennzeichnet, dass** der Korrekturfaktor vorab in vivo durch Messung der Substanz-Konzentration im Blut bestimmt wird.

5. Verfahren nach einem der Ansprüche 1 bis 4, **dadurch gekennzeichnet, dass** zur Erzielung hoher Durchflussraten die Spülflüssigkeit oder das Gemisch Spülflüssigkeit/Körperflüssigkeit zum und aus dem Körper gepumpt wird.

6. Verfahren nach einem der Ansprüche 1 bis 5, **dadurch gekennzeichnet, dass** als Referenzsubstanzionen Natrium-Ionen, Kalium-Ionen und/oder Chlorid-Ionen verwendet werden.

7. Vorrichtung zur Messung der Konzentration von Substanzen in lebenden Organismen mittels Mikrodialyse, mit einem Mikrodialyse-Katheter (3), der eine Membran (26) aufweist, durch die hindurch Körperflüssigkeit mit einer von einem Perfusat-Reservoir (1) zugeführten Spülflüssigkeit vermisch bar ist, und mit einer Sensoreinheit (4) sowie daran angeschlossen einer elektronischen Answerteinheit (8) zur Bestimmung der Substanz-Konzentration der Körperflüssigkeit, **dadurch gekennzeichnet, dass** die Membran (26) auch für den Durchtritt von Ionen als endogene Referenzsubstanz mit bekannter Konzentration eingerichtet ist, dass die Sensoreinheit (4) zur Messung dieser Referenzionen nach Membran-Durchtritt und nach Vermischen mit der Spülflüssigkeit auf Basis der elektrischen Leitfähigkeit eingerichtet ist, und dass die elektronische Auswerteinheit (8) eingerichtet ist, die tatsächliche Substanz-Konzentration auf Basis des Quotienten aus bekannter Referenzionen-Konzentration durch gemessene Referenzionen-Konzentration, multipliziert mit der mit Hilfe der Sensoreinheit (4) bestimmten Substanz-Konzentration, zu ermitteln.

8. Vorrichtung nach Anspruch 7, **dadurch gekennzeichnet, dass** sowohl im Zulauf (24') als auch im Ablauf (25') des Mikrodialyse-Katheters (3) eine Pumpeinheit (2, 5) angeordnet ist.

9. Vorrichtung nach Anspruch 8, **dadurch gekennzeichnet, dass** die beiden Pumpeinheiten (2, 5) synchronisiert sind.

10. Vorrichtung nach Anspruch 8 oder 9, **dadurch gekennzeichnet, dass** die Ablauf-seitige Pumpeinheit (5) in Strömungsrichtung gesehen hinter der Sensoreinheit (4) angeordnet ist.

11. Vorrichtung nach einem der Ansprüche 8 bis 10, **dadurch gekennzeichnet, dass** die beiden Pumpeinheiten (2, 5) durch Peristaltikpumpen gebildet sind.

12. Vorrichtung nach einem der Ansprüche 8 bis 11, **dadurch gekennzeichnet, dass** die Sensoreinheit (4) zur Bestimmung der Leitfähigkeit der durchströmenden Flüssigkeit Elektroden (46, 47, 48, 49; 42, 43) aufweist.

**Claims**

1. Method for measuring the concentration of substances in living organisms using microdialysis, wherein bodily fluid is passed through a membrane and mixed with a rinsing fluid, and a substance concentration of the bodily fluid is determined using a sensor unit, **characterized in that** in order to take into consideration the restricted transport of the substance through the membrane, so that the measured substance concentration differs from the actual substance concentration in the organism, ions in known concentration are additionally measured after passing through the membrane and mixing with the rinsing fluid, as an endogenous reference substance, on the basis of the measurement of electrical conductivity corresponding to the reference ions and the actual substance concentration is ascertained on the basis of the quotient of the known reference ion concentration divided by the measured reference ion concentration, multiplied by the determined substance concentration.

**2.** Method according to Claim 1, **characterized in that** any difference in transport behaviour between the reference ions substance and the substance to be measured is taken into consideration by means of a correction factor that is determined in advance.

**3.** Method according to Claim 2, **characterized in that** the correction factor is determined in advance, in vitro, by preparing a solution with ions and the substance to be determined, and passing it through a membrane.

**4.** Method according to Claim 2, **characterized in that** the correction factor is determined in advance, in vivo, by measuring the substance concentration in the blood.

**5.** Method according to one of Claims 1 to 4, **characterized in that** in order to achieve high flow-through rates, the rinsing fluid or the mixture of rinsing fluid/bodily fluid is pumped into and out of the body.

**6.** Method according to one of Claims 1 to 5, **characterized in that** sodium ions, potassium ions and/or chloride ions are used as reference substance ions.

**7.** Device for measuring the concentration of substances in living organisms using microdialysis, having a microdialysis catheter (3) that has a membrane (26), through which bodily fluid is mixable with a rinsing fluid that is supplied from a perfusate reservoir (1), and having a sensor unit (4) and an electronic evaluation unit (8) connected thereto for determining the substance concentration of the bodily fluid, **characterized in that** the membrane (26) is also arranged for passage of ions in known concentration as an endogenous reference substance, that the sensor unit (4) is arranged for measuring these reference ions on the basis of electrical conductivity after passing through the membrane and mixing with the rinsing fluid, and that the electronic evaluation unit (8) is arranged to ascertain the actual substance concentration on the basis of the quotient of the known reference ion concentration divided by the measured reference ion concentration, multiplied by the substance concentration determined using the sensor unit (4).

**8.** Device according to Claim 7, **characterized in that** a pump unit (2, 5) is arranged both in the inflow (24') and the outflow (25') of the microdialysis catheter (3).

**9.** Device according to Claim 8, **characterized in that** the two pump units (2, 5) are synchronized.

**10.** Device according to Claim 8 or 9, **characterized in that** the pump unit (5) on the outflow side is arranged downwardly of the sensor unit (4), viewed in the flow direction.

**11.** Device according to one of Claims 8 to 10, **characterized in that** the two pump units (2, 5) are formed by peristaltic pumps.

**12.** Device according to one of Claims 8 to 11, **characterized in that** the sensor unit (4) has electrodes (46, 47, 48, 49; 42, 43) for determining the conductivity of the fluid that flows through.

## Revendications

**1.** Procédé pour mesurer la concentration de substances dans des organismes vivants par microdialyse, où un liquide biologique est mélangé avec un liquide de rinçage à travers une membrane et une concentration de substance du liquide biologique est déterminée à l'aide d'une unité de détection, **caractérisé en ce que**, pour tenir compte du transport limité de la substance à travers la membrane, de sorte que la concentration de substance mesurée est différente de la concentration de substance réelle dans l'organisme, en complément des ions de concentration connue à titre de substance de référence endogène sont mesurés après traversée de la membrane et mélange avec le liquide de rinçage sur la base de la mesure de la conductivité électrique conformément aux ions de référence et la concentration de substance réelle est déterminée sur la base du quotient de la concentration d'ions de référence connue et de la concentration d'ions de référence mesurée, multiplié par la concentration de substance déterminée.

**2.** Procédé selon la revendication 1 **caractérisé en ce qu'**un comportement de transport des ions de référence et de la substance à mesurer éventuellement différent est pris en compte par un facteur de correction déterminé au préalable.

3. Procédé selon la revendication 2 **caractérisé en ce que** le facteur de correction est déterminé au préalable in vitro en préparant une solution avec des ions et la substance à déterminer et en la faisant passer à travers une membrane.

4. Procédé selon la revendication 2 **caractérisé en ce que** le facteur de correction est déterminé au préalable in vivo en mesurant la concentration de substance dans le sang.

5. Procédé selon l'une des revendications 1 à 4 **caractérisé en ce que**, pour obtenir des débits importants, le liquide de rinçage ou le mélange liquide de rinçage/liquide biologique est pompé vers le corps et depuis le corps.

6. Procédé selon l'une des revendications 1 à 5 **caractérisé en ce que** des ions sodium, des ions potassium et/ou des ions chlorure sont utilisés comme ions de substance de référence.

7. Dispositif pour mesurer la concentration de substances dans des organismes vivants par microdialyse, avec un cathéter de microdialyse (3), qui comporte une membrane (26) à travers laquelle un liquide biologique peut être mélangé avec un liquide de rinçage amené depuis un réservoir de perfusat (1), et avec une unité de détection (4) ainsi qu'une unité d'exploitation électronique (8) raccordée à celle-ci pour la détermination de la concentration de substance du liquide biologique, **caractérisé en ce que** la membrane (26) est agencée aussi pour la traversée d'ions de concentration connue à titre de substance de référence endogène, **en ce que** l'unité de détection (4) est agencé pour la mesure de ces ions de référence après la traversée de la membrane et après le mélange avec le liquide de rinçage sur la base de la conductivité électrique, et **en ce que** l'unité d'exploitation électronique (8) est agencée pour déterminer la concentration de substance réelle sur la base du quotient de la concentration d'ions de référence connue et de la concentration d'ions de référence mesurée, multiplié par la concentration de substance déterminée à l'aide de l'unité de détection (4).

8. Dispositif selon la revendication 7 **caractérisé en ce qu'**une unité de pompage (2, 5) est disposée dans l'alimentation (24') ainsi que dans l'évacuation (25') du cathéter de microdialyse (3).

9. Dispositif selon la revendication 8 **caractérisé en ce que** les deux unités de pompage (2, 5) sont synchronisées.

10. Dispositif selon la revendication 8 ou 9 **caractérisé en ce que** l'unité de pompage (5) du côté évacuation est disposée, vue dans le sens de l'écoulement, derrière l'unité de détection (4).

11. Dispositif selon l'une des revendications 8 à 10 **caractérisé en ce que** les deux unités de pompage (2, 5) sont formées par des pompes péristaltiques.

12. Dispositif selon l'une des revendications 8 à 11 **caractérisé en ce que** l'unité de détection (4) comporte des électrodes (46, 47, 48, 49 ; 42, 43) pour la détermination de la conductivité du liquide traversant.

Fig. 1

Fig. 2

Fig. 3

Fig. 4

Fig. 5

Fig. 6

EP 1 404 217 B1

Fig. 7

Fig. 8

Fig. 8A

Fig. 9

Fig. 10

Fig. 11

EP 1 404 217 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5191900 A
- US 4694832 A
- EP 300008 B
- US 5193545 A
- US 5002054 A
- US 5954643 A
- EP 0300008 A
- WO 9635369 A1
- DE 19715440 C1
- EP 1072222 A2
- WO 9305701 A
- GB 2297383 A
- EP 0300008 B
- US 5097834 A
- US 5706806 A